# EUROPEAN PATENT SPECIFICATION

(11) **EP 1 463 461 B1**
(45) Date of publication and mention of the grant of the patent: **30.07.2014**
(21) Application number: 03729571.4
(22) Date of filing: 03.01.2003
(51) Int. Cl.: A61F 2/91, A61F 2/915

(54) **STENT**
STENT
STENT

(30) Priority: 09.01.2002 US 42634
(43) Date of publication of application: 06.10.2004
(73) Proprietor: Boston Scientific Limited, St. Michael, Barbados, West Indies (BB)
(72) Inventor: GIRTON, Timothy, Minneapolis, MN 55419 (US); GREGORICH, Daniel, Mound, MN 55364 (US)
(74) Representative: Hauck Patent- und Rechtsanwälte
(86) International application number: PCT/US2003/000179
(87) International publication number: WO 2003/059207

(56) References cited:
- WO-A-00/30563
- US-A1- 2001 020 183

## Description

### BACKGROUND OF THE INVENTION

The use of stents in bodily lumen is well known. A stent is typically delivered in an unexpanded state to a desired location in a bodily lumen and then expanded. The stent may be expanded via the use of mechanical device such as a balloon or the stent may be self-expanding.

Because a stent often must be delivered through tortuous anatomy, it is desirable for the stent to be flexible. Increased flexibility in a stent, however, typically comes at the expense of scaffolding strength. Moreover, design features which may result in increased flexibility may also result in protruding edges which may damage vessels walls or catheter balloons during delivery of the stent through tortuous vasculature.

Many stents of conventional design include a plurality of serpentine bands which define openings in the sidewall of the stent. Typically, the openings are parallel to the longtudinal axis of the stent. Stents have been produced with openings which are oblique relative to the longitudinal axis of the stent. Stents where all of the openings are parallel to one another, however, may experience excessive torque upon delivery through tortuous vessels and resultant deployment problems.

US 2001/020183 A1 discloses a tubular stent that consists of chevron-shape expansion struts and contralaterally attached diagonal-connectors. WO 00/30563 A discloses a segmented articulatable stent of open structure comprised of end-connected struts of first and second length making up first and second segments with angular interconnects between adjacent first and second segments.

There remains a need for a stent which has a high degree of flexibility in the unexpanded state, has adequate scaffolding strength and which does not experience excessive torque on delivery.

Without limiting the scope of the invention, a brief summary of the claimed embodiments of the invention is set forth below. Additional details of the summarized embodiments of the invention and/or additional embodiments of the invention may be found in the Detailed Description of the Invention below.

### SUMMARY OF THE INVENTION

In one embodiment, the invention is directed to a stent comprising a plurality of circumferential bands, circumferential bands which are adjacent one another connected one to the other, the stent including first circumferential bands characterized by a first number of alternating first peaks and first troughs joined by bent struts and second circumferential bands characterized by a second number of alternating second peaks and second troughs joined by bent struts, the second number different from the first number.

The first and second circumferential bands each define a pathway around the periphery of the stent. The first and second pathways may be of the same length or of different lengths.

Desirably, the first and second peaks and first and second troughs are oriented at an angle between 0° and 70° with respect to the longitudinal axis of the stent. More desirably, the first and second peaks and first and second troughs are oriented at an angle of at least 10 degrees with respect to the longitudinal axis of the stent and most desirably, the first and second peaks and first and second troughs are oriented at an angle of at least 15 degrees with respect to the longitudinal axis of the stent.

Typically, the first and second circumferential bands may be characterized by a longitudinal extent with the longitudinal extent of each first circumferential band desirably exceeding the longitudinal extent of each second circumferential band.

Optionally, each of the bent struts may be characterized by a width with the width of the bent struts of the first bands exceeding the width of the bent struts of the second bands.

Desirably, bent struts which are circumferentially adjacent one another are parallel to one another. More desirably, bent struts in longitudinally adjacent first and second circumferential bands are non-parallel to one another.

In one embodiment of the invention, first and second circumferential bands which are longitudinally adjacent to one another are connected by at least one connector and desirably, by a plurality of connectors. Typically, the connectors will be straight and non-parallel to the longitudinal axis of the stent. Desirably, the connectors extend from peaks of circumferential bands to troughs of adjacent circumferential bands. Also desirably, the connectors are shorter in length than the longitudinal extent of the second circumferential bands.

Where a plurality of connectors are present between adjacent first and second circumferential bands, circumferentially adjacent connectors are joined via a first pathway along a first circumferential band and a second pathway along a second circumferential band. The first pathway is desirably of the same length as the second pathway.

In one embodiment, each first pathway traverses a total of three peaks and troughs (i.e. two peaks and one trough or one peak and two troughs) and each second pathway traverse a total of five peaks and troughs (i.e. three peaks and two troughs or two peaks and three troughs).

In yet another embodiment, the invention is directed to a stent comprising a plurality of circumferential bands where circumferential bands which are adjacent one another are connected one to the other. The circumferential bands include first circumferential bands characterized by a first number of alternating first peaks and first troughs and second circumferential bands characterized by a second number of alternating second peaks and second troughs. The second number is different from the first number. The first peaks and troughs are oriented non-parallel to the longitudinal axis of the stent and the second peaks and second troughs are oriented non-parallel to the longitudinal axis of the stent. Optionally, the first and second circumferential bands each define a pathway around the periphery of the stent and the first and second pathways are the same length.

Desirably, the peaks and troughs are oriented at an angle of at least 10 degrees with respect to the longitudinal axis of the stent. More desirably, the peaks and troughs are oriented at an angle of at least 15 degrees with respect to the longitudinal axis of the stent.

Desirably, the first and second circumferential bands are each characterized by a longitudinal extent with the longitudinal extent of the first circumferential bands exceeding the longitudinal extent of the second circumferential bands.

Also desirably, first peaks and first troughs which are circumferentially adjacent one another are connected by struts and second peaks and second troughs which are circumferentially adjacent one another are connected by struts. Each of the struts is characterized by a width. The width of the struts of the first bands exceeds the width of the struts of the second bands. Typically, struts which are circumferentially adjacent one another are parallel to one another.

First and second circumferential bands which are longitudinally adjacent one another may be connected by a single connector or by a plurality of connectors. The connectors may be of any shape. In one embodiment, straight connectors are used. The connectors may be oriented parallel to the longitudinal axis or, in another embodiment, non-parallel to the longitudinal axis. Connectors with curved portions may also be used.

The connectors may extend from any region of one band to any region of an adjacent band. In one embodiment, the connectors extend from peaks of circumferential bands to troughs of adjacent circumferential bands. In one desirable embodiment, first and second circumferential bands which are longitudinally adjacent one another are connected by a plurality of connectors and the connectors are shorter in length than the longitudinal extent of the second circumferential bands. Circumferentially adjacent connectors may be joined via a first pathway along a first circumferential band and a second pathway along a second circumferential band with the first pathway being of the same length as the second pathway.

Desirably, the struts in first bands which are longitudinally adjacent one another are non-parallel to one another. More desirably, the struts in first bands which are longitudinally adjacent one another slant in opposing directions relative to the longitudinal axis of the stent.

In yet another embodiment of the invention, the first circumferential bands are connected to the second circumferential bands via straight connectors which extend between portions of similar curvature on adjacent circumferential bands. Desirably, the connectors extend between peaks of first circumferential bands and peaks of second circumferential bands and between troughs of second circumferential bands and troughs of first circumferential bands.

Typically, the connectors are shorter in length than the longitudinal extent L₁ of first circumferential bands.

In another embodiment, the invention is directed to a stent comprising a sidewall with a plurality of openings therein. Each opening is bounded by at least a first stent member and a second stent member. The first stent member is of a larger width than the second stent member. The first stent member comprises a plurality of bent first struts which extend non-parallel to the longitudinal axis of the stent and the second stent member comprises a plurality of bent second struts which extend non-parallel to the longitudinal axis of the stent. The bent first struts define finger like first projections which are non-parallel to the longitudinal axis of the stent and the bent second struts define finger like second projections which are non-parallel to the longitudinal axis of the stent with the number of first projections exceeding the number of second projections.

In one embodiment, each opening is defined by first projections which are non-parallel to the second projections.

In another embodiment, the openings comprise first openings and second openings, with each first opening defined by first projections which are parallel to the second projections. Typically each second opening is defined by first projections which are non-parallel to second projections.

In another embodiment, the invention is directed to a stent comprising a sidewall, the sidewall having a plurality of openings therein. Each opening is bounded by at least a first stent member and a second stent member. The first stent member is of a larger width than the second stent member. The first stent member comprises a plurality of bent first struts which extend non-parallel to the longitudinal axis of the stent and the second stent member comprises a plurality of bent second struts which extend non-parallel to the longitudinal axis of the stent. The bent first struts define finger like first projections which are non-parallel to the longitudinal axis of the stent and the bent second struts define finger like second projections which are non-parallel to the longitudinal axis of the stent. The number of first projections exceeds the number of second projections.

Without being bound by theory, bent struts have been found to provide more wall coverage than straight struts. Furthermore, using bent struts typically requires more material, i.e. metal, and thus provides improved radiopacity as well.

Additional details and/or embodiments of the invention are discussed below.

### BRIEF DESCRIPTION OF THE FIGURES

- Fig. 1a: is a flat layout view of an inventive stent.
- Fig. 1b: shows an enlarged view of region A of the stent shown in flat layout view in Fig. 1a.
- Fig. 2a: is a flat layout view of an embodiment of the inventive stent of the present invention which is similar to that shown in Fig. 1a.
- Fig. 2b: shows an enlarged view of region A of the stent shown in flat layout view in Fig. 2a.
- Fig. 3a: shows an alternative embodiment of the inventive stent of the present invention.
- Fig. 3b: shows an enlarged view of region A of the stent shown in flat layout view in Fig. 3 a.
- Fig. 4a: is a flat layout view of an embodiment of the inventive stent of the present invention.
- Fig. 4b: shows an enlarged view of region A of the stent shown in flat layout view in Fig. 4a.
- Fig. 5a: is a flat layout view of an example stent.
- Fig. 5b: shows an enlarged view of region A of the stent shown in flat layout view in Fig. 5 a.
- Fig. 6a: is a flat layout view of an embodiment of the inventive stent of the present invention.
- Fig. 6b: shows an enlarged view of region A of the stent shown in flat layout view in Fig. 6a.
- Fig. 7a: is a flat layout view of an embodiment of the inventive stent of the present invention.
- Fig. 7b: shows an enlarged view of region A of the stent shown in flat layout view in Fig. 7a.
- Fig. 8a: is a flat layout view of an embodiment of the inventive stent of the present invention.
- Fig. 8b: shows an enlarged view of region A of the stent shown in flat layout view in Fig. 8a.
- Fig. 9a: is a flat layout view of an inventive stent of the present invention.
- Fig. 9b: shows an enlarged view of region A of the stent shown in flat layout view in Fig. 9a.
- Fig. 10a: is a flat layout view of an inventive stent of the present invention.
- Fig. 10b: shows an enlarged view of region A of the stent shown in flat layout view in Fig. 10a.
- Fig. 11: shows a flat layout view of an alternative embodiment of the inventive stent of the present invention.

### DETAILED DESCRIPTION

While this invention may be embodied in many different forms, there are described in detail herein specific embodiments of the invention. This description is an exemplification of the principles of the invention and is not intended to limit the invention to the particular embodiments illustrated.

For the purposes of this disclosure, like reference numerals in the figures shall refer to like features unless otherwise indicated.

Also for the purposes of this disclosure, the term 'bent strut' does not implicate a method of manufacture and is intended to include struts which have curves, struts which are angled, and struts which are curvilinear, regardless of how the struts or the stent as a whole are manufactured.

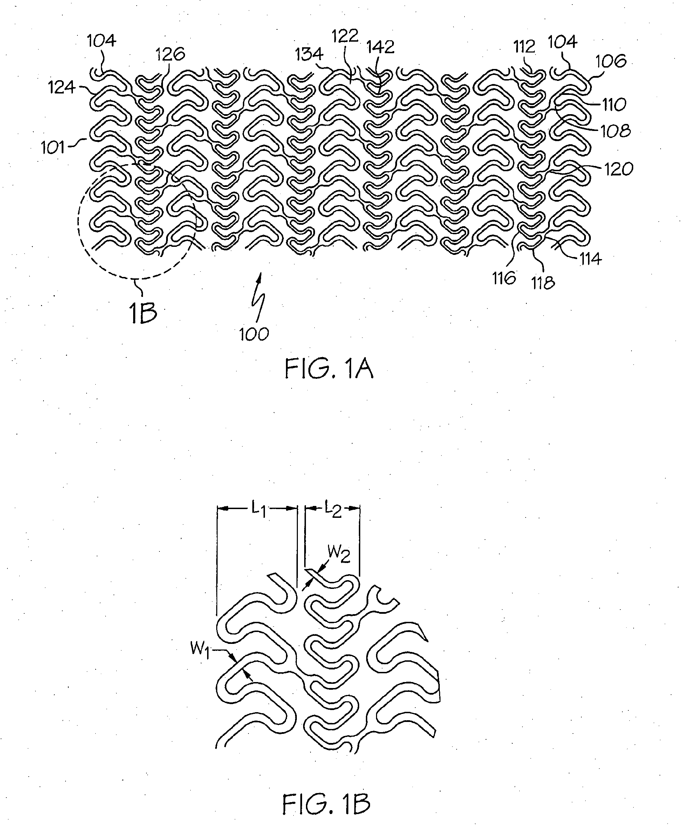

In one embodiment, the invention is directed to a stent such as that shown by way of example at 100 in Fig. 1a comprising a plurality of circumferential bands. Circumferential bands which are adjacent one another are connected one to the other. The circumferential bands include first circumferential bands 104 characterized by a first number of alternating first peaks 106 and first troughs 108 joined by bent struts 110 and second circumferential bands 112 characterized by a second number of alternating second peaks 114 and second troughs 116 joined by bent struts, 118. Typically, as shown in Fig. 1a, the second number of second peaks and troughs is different from the first number of first peaks and troughs and desirably exceeds the first number.

The first and second circumferential bands each define a pathway around the periphery of the stent. The first and second pathways may be of the same length or of different lengths. Desirably, the first and second pathways are the same length.

Also desirably, the first and second peaks and first and second troughs are oriented at an angle between 0° and 70° with respect to the longitudinal axis of the stent, more desirably they are oriented at an angle of at least 10 degrees with respect to the longitudinal axis of the stent, and most desirably, the first and second peaks and first and second troughs are oriented at an angle of at least 15 degrees with respect to the longitudinal axis of the stent.

Typically, as shown in Fig. 1b, the first and second circumferential bands may be characterized by longitudinal extents L₁ and L₂. The longitudinal extent of each first circumferential band L₁ desirably exceeds the longitudinal extent L₂ of the individual second circumferential bands.

Each of the bent struts may be characterized by a width. Optionally, the width of the bent struts of the first bands W₁ exceeds the width of the bent struts of the second bands W₂.

Desirably, as shown in Fig. 1a, bent struts which are circumferentially adjacent one another are parallel to one another. More desirably, as shown in Fig. 1a, bent struts in longitudinally adjacent first and second circumferential bands are non-parallel to one another.

In one embodiment of the invention, as shown in Fig. 1a, first and second circumferential bands which are longitudinally adjacent one another are connected by at least one connector 120 and desirably, by a plurality of connectors. Typically, the connectors will be straight and non-parallel to the longitudinal axis 101 of the stent. In other embodiments of the invention, other types of connectors may be used - for example connectors with one or more curves and/or connectors of different lengths. Desirably, as shown in Fig. 1a, the connectors extend from peaks of circumferential bands to troughs of adjacent circumferential bands. Also desirably, as shown in Fig. 1a, the connectors are shorter in length than the longitudinal extent L₂ of the second circumferential bands.

Where a plurality of connectors are present between adjacent first and second circumferential bands, circumferentially adjacent connectors are joined via a first pathway along a first circumferential band and a second pathway along a second circumferential band, the first pathway desirably being of the same length as the second pathway.

In the embodiment of Fig. 1a, each first pathway traverses a total of three peaks and troughs (i.e. two peaks and one trough or one peak and two troughs) and each second pathway traverse a total of five peaks and troughs (i.e. three peaks and two troughs or two peaks and three troughs).

In other words, between circumferentially adjacent connectors which connect first and second circumferential bands together, in the first band, there are a total of three peaks and troughs between the connectors, and in the second circumferential band there are a total of five peaks and troughs between connectors.

Without being bound by theory, the alternating orientation of adjacent first and second circumferential bands is believed to prevent significant rotation and build-up of torque and the accompanying degradation of stent performance.

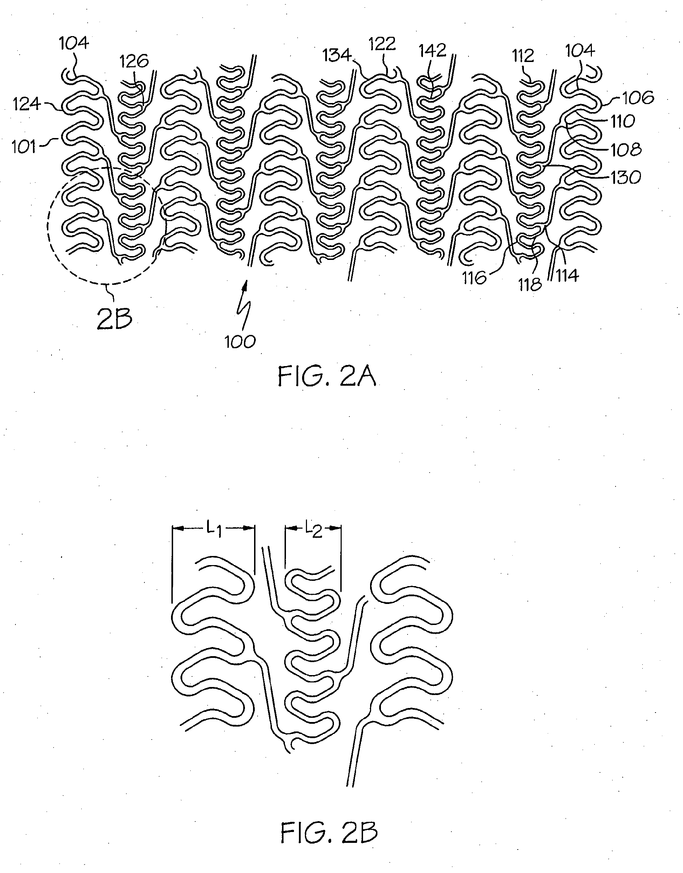

In another embodiment, the invention is directed to a stent such as that shown by way of example at 100 in Fig. 2a comprising a plurality of circumferential bands substantially similar to those shown in Fig. 1a. As in the embodiment shown in Fig. 1a, the first and second circumferential bands each define a pathway around the periphery of the stent. The first and second pathways may be of the same length or of different lengths. Desirably, the first and second pathways are the same length.

Also as in the embodiment shown in Fig. 1a, the first circumferential bands 104 are characterized by a first number of alternating first peaks 106 and first troughs 108 joined by bent struts 110 and second circumferential bands 112 characterized by a second number of alternating second peaks 114 and second troughs 116 joined by bent struts, 118. Typically, as shown in Fig. 1a, the second number of second peaks and troughs is different from the first number of first peaks and troughs and desirably exceeds the first number.

In the embodiment shown in Fig. 2a, however, in contrast to that shown in Fig. 1a, the connectors do not extend between the nearest neighboring peaks and troughs, but rather every first peak of every first circumferential band 104 is connected to every third trough of a second adjacent circumferential band.

As in Fig. 1b, the first and second circumferential bands may be characterized by longitudinal extents L₁ and L₂ as shown in Fig. 2b. The longitudinal extent of each first circumferential band L₁ desirably exceeds the longitudinal extent L₂ of the individual second circumferential bands.

Again as in Fig. 1a, the first and second circumferential bands which are longitudinally adjacent one another are connected by at least one connector 120 and desirably, by a plurality of connectors. Desirably, as shown in Fig. 2a, the connectors extend from peaks of circumferential bands to troughs of adjacent circumferential bands as in Fig. 1a. However, in the embodiment shown in Fig. 2a, the connectors are significantly longer in length than the longitudinal extent L₂ of the second circumferential bands.

Where a plurality of connectors are present between adjacent first and second circumferential bands, circumferentially adjacent connectors are joined via a first pathway along a first circumferential band and a second pathway along a second circumferential band, the first pathway desirably being of the same length as the second pathway.

In the embodiment shown in Fig. 2a, each first pathway traverses a total of four peaks and troughs (i.e. two peaks and two troughs) and each second pathway traverse a total of six peaks and troughs (i.e. three peaks and three troughs).

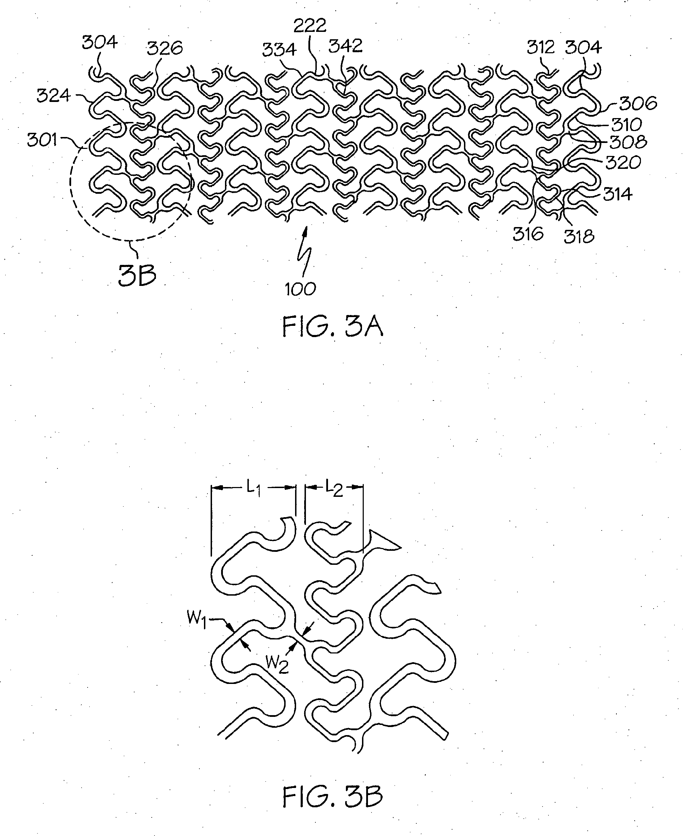

In another embodiment, the invention is directed to a stent such as that shown by way of example at 300 in Fig. 3a comprising a plurality of circumferential bands. This embodiment is also similar to the embodiments shown in Fig. 1a and 2a. Circumferential bands which are adjacent one another are connected one to the other. The circumferential bands include first circumferential bands 304 characterized by a first number of alternating first peaks 306 and first troughs 308 joined by bent struts 310 and second circumferential bands 312 characterized by a second number of alternating second peaks 314 and second troughs 316 joined by bent struts. The number of second peaks and troughs is different from the number of first peaks and troughs and desirably exceeds the first number.

The first and second circumferential bands each define a pathway around the periphery of the stent. The first and second pathways may be of the same length or of different lengths but are desirably the same length.

As shown in Fig. 3b, the first and second troughs are oriented at an angle of at least 10 degrees with respect to the longitudinal axis 301 of the stent. More desirably, the first and second peaks and first and second troughs are oriented at an angle of at least 15 degrees with respect to the longitudinal axis of the stent. In the particular embodiment shown in Fig. 3b, the first and second peaks and first and second troughs, are actually oriented at an angle of about 40 degrees with respect to the longitudinal axis of the stent.

Typically, as shown in Fig. 3b, the first and second circumferential bands may be characterized by longitudinal lengths L₁ and L₂ which may be of the same length, or of a different length. Desirably, L₁ exceeds the longitudinal extent L₂ of the individual second circumferential bands.

Each of the bent struts may be characterized by a width. Optionally, the width of the bent struts of the first bands W₁ exceeds the width of the bent struts of the second bands W₂.

The first and second circumferential bands which are longitudinally adjacent one another are connected by at least one connector 320 and preferably by a plurality of connectors. In this particular embodiment, the connectors are straight, and are non-parallel to the longitudinal axis 301 of the stent 300. Also in the embodiment shown in Fig. 3a, the connectors are significantly shorter than the longitudinal extent of the circumferential bands L₂. Other connectors may be optionally used, however including connectors having more curves or being of a different length.

In this embodiment, the connectors extend between peaks of circumferential bands to troughs of adjacent circumferential bands. In this particular embodiment, the connectors are shorter in length than the extent L₂ of the second circumferential bands.

This may be optionally described in terms of first and second pathways. Where a plurality of connectors are present between adjacent first and second circumferential bands, circumferentially adjacent connectors are joined via a first pathway along a first circumferential band and a second pathway along a second circumferential band, the first pathway desirably being of the same length as the second pathway.

In the embodiment shown in Fig. 3a, the first pathway traverses a total of four peaks and troughs (two peaks and two troughs) and the second pathway traverses a total of six peaks and troughs (three peaks and three troughs).

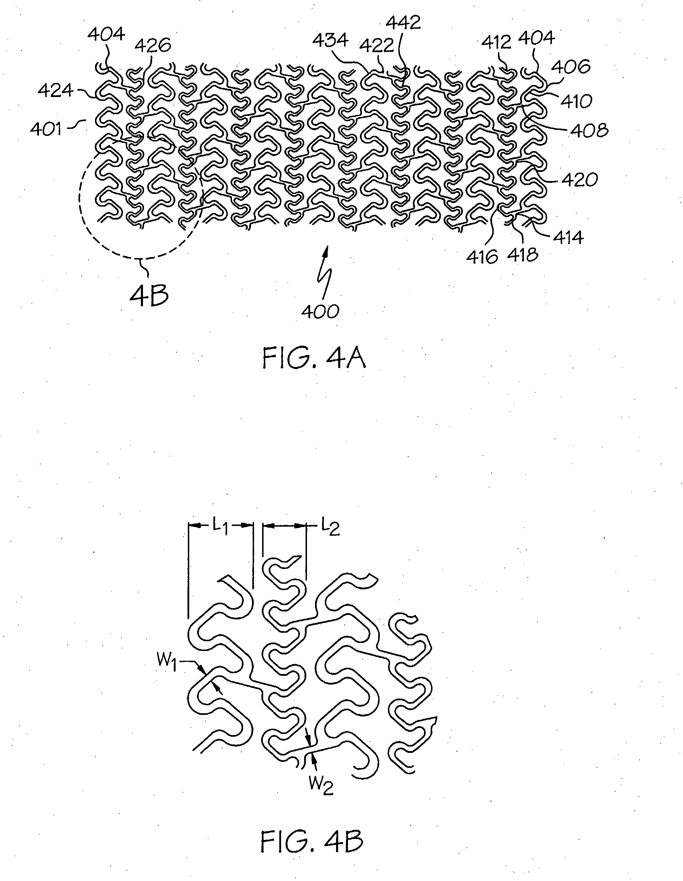

Fig. 4a illustrates an embodiment similar to those shown in Figs. 1a-3a as described above.

Again, there arc first and second circumferential bands which define a pathway around the periphery of the stent. The first and second pathways may be of the same length or of different lengths but are desirably the same length.

The first and second circumferential bands which are longitudinally adjacent one another are connected by at least one connector 420 and preferably by a plurality of connectors. In this particular embodiment, the connectors are straight, and are non-parallel to the longitudinal axis 401 of the stent 400. Other connectors may be optionally used, however including connectors having more curves or being of a different length.

In this embodiment, the connectors extend between peaks of circumferential bands to troughs of adjacent circumferential bands. In this particular embodiment, the connectors are shorter in length than the extent L₂ of the second circumferential bands.

This may be optionally described in terms of first and second pathways. Where a plurality of connectors are present between adjacent first and second circumferential bands, circumferentially adjacent connectors are joined via a first pathway along a first circumferential band and a second pathway along a second circumferential band, the first pathway desirably being of the same length as the second pathway.

Again, as in the embodiment shown in Fig. 3a, the embodiment shown in Fig. 4a includes a first pathway which traverses a total of four peaks and troughs (two peaks and two troughs) and the second pathway traverses a total of six peaks and troughs (three peaks and three troughs).

The connectors in the embodiment shown in Fig. 4a are at a different angle relative to the longitudinal axis 401 of stent 400 than those shown in Fig. 3a, the angle being smaller relative to the longitudinal axis in the embodiment shown in Fig. 4a.

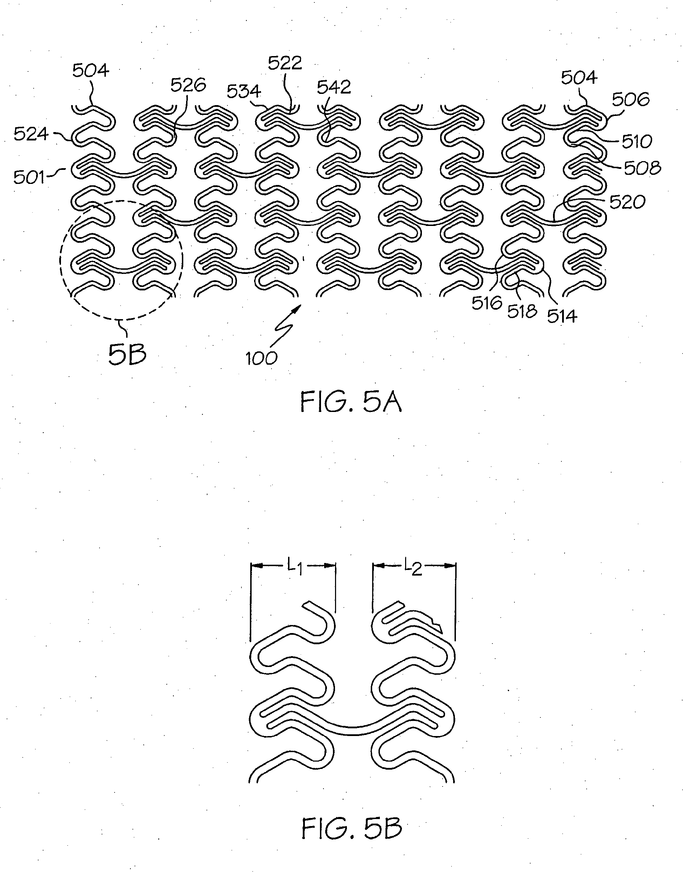

An example of a stent is shown generally at 500 in Fig. 5a and again includes a plurality of circumferential bands. Circumferential bands which are adjacent one another arc connected one to the other. The circumferential bands include first circumferential bands 504 characterized by a first number of alternating first peaks 506 and first troughs 508 joined by bent struts 510 and second circumferential bands 512 characterized by a second number of alternating second peaks 514 and second troughs 516 joined by bent struts 518. In this particular example stent, the number of second peaks and troughs is the same as the number of first peaks and troughs.

The first and second circumferential bands each define a pathway around the periphery of the stent. The first and second pathways may be of the same length or of a different length. In the embodiment shown in Fig. 5a, the first and second pathways are of the same length.

The first and second peaks and first and second troughs are oriented at an angle of at least 10 degrees with respect to the longitudinal axis 501 of the stent 500, and desirably are oriented at an angle of at least 15 degrees with respect to the longitudinal axis 501.

In this particular example stent, as shown in Fig. 5b, the longitudinal extent L₁ of the first circumferential band is substantially equal in length to the longitudinal extent L₂ of the second circumferential band.

Also in the example stent shown in Fig. 5b, the width of each of the bent struts of the first bands W₁ is equal to the width of the bent struts of the second bands W₂. Optionally, the widths may be different. For example, the width of the first bands W₁ may exceed the width of the second bands W₂.

In the example stent shown in Fig. 5a, first and second circumferential bands which arc longitudinally adjacent one another are connected by at least one connector 520 and desirably, by a plurality of connectors. Typically, in this example stent, the connectors will be curved as opposed to the straight conncctors as shown in some of the other embodiments. In this example stent, the connectors extend from the troughs of circumferential bands to troughs of adjacent circumferential bands. The connectors are shown longer in length than the longitudinal extents L₁ and L₂ of the circumferential bands.

Where a plurality of connectors are present between adjacent first and second circumferential bands, circumferentially adjacent connectors are joined via a first pathway along a first circumferential band and a second pathway along a second circumferential band, the first pathway desirably being of the same length as the second pathway. Each first pathway traverses a total of six peaks and troughs (three peaks and three troughs) and each second pathway traverses a total of six peaks and troughs (three peaks and three troughs).

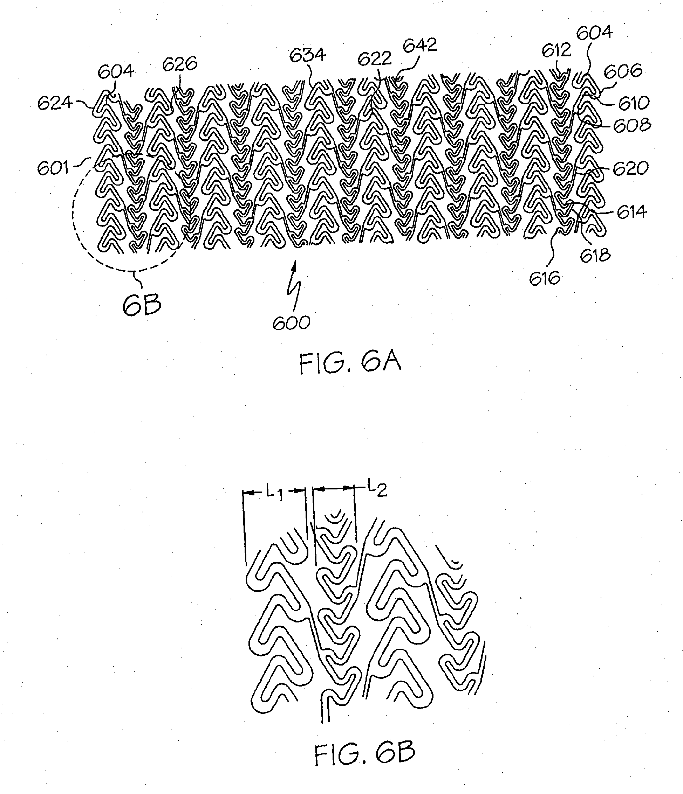

In yet another alternative embodiment of the inventive stent of the present invention shown generally at 600 in Fig. 6a, the stent includes a plurality of circumferential bands wherein circumferential bands which are adjacent one another are connected one to the other. The circumferential bands include first circumferential bands 604 characterized by a first number of alternating first peaks 606 and first troughs 608 and second circumferential bands 612 characterized by a second number of alternating second peaks 614 and second troughs 616. The second number is different from the first number. The first peaks and troughs are oriented non-parallel to the longitudinal axis 601 of the stent 600 and the second peaks and second troughs are oriented non-parallel to the longitudinal axis 601 of the stent. Desirably, the peaks and troughs are oriented at an angle of at least 10 degrees with respect to the longitudinal axis of the stent. More desirably, the peaks and troughs are oriented at an angle of at least 15 degrees with respect to the longitudinal axis of the stent.

The first and second circumferential bands each define a pathway around the periphery of the stent and the first and second pathways are the same length.

The stent is further characterized by bent struts which exhibit a configuration similar to finger-like projections. Each of the bent struts may be characterized by a width. Optionally, the width of the bent struts of the first bands W₁ exceeds the width of the bent struts of the second bands W₂.

Desirably, as shown in Fig. 6a, bent struts which are circumferentially adjacent one another are parallel to one another. Bent struts in longitudinally adjacent first and second circumferential bands may or may not be parallel to one another, however.

In the embodiment shown in Fig. 6a, first and second circumferential bands which are longitudinally adjacent one another are connected by at least one connector 620 and desirably, by a plurality of connectors. Typically, the connectors will be substantially straight and non-parallel to the longitudinal axis 601 of the stent. In other embodiments of the invention, other types of connectors may be used - for example connectors with one or more curves and/or connectors of different lengths. Desirably, as shown in Fig. 6a, the connectors extend from peaks of circumferential bands to troughs of adjacent circumferential bands. Also desirably, as shown in Fig. 6a, the connectors are similar in length to the longitudinal extent L₂ of the second circumferential bands.

Where a plurality of connectors are present between adjacent first and second circumferential bands, circumferentially adjacent connectors are joined via a first pathway along a first circumferential band and a second pathway along a second circumferential band, the first pathway desirably being of the same length as the second pathway.

In the embodiment of Fig. 6a, each first pathway traverses a total of four peaks and troughs (i.e. two peaks and two troughs) and each second pathway traverse a total of six peaks and troughs (i.e. three peaks and three troughs).

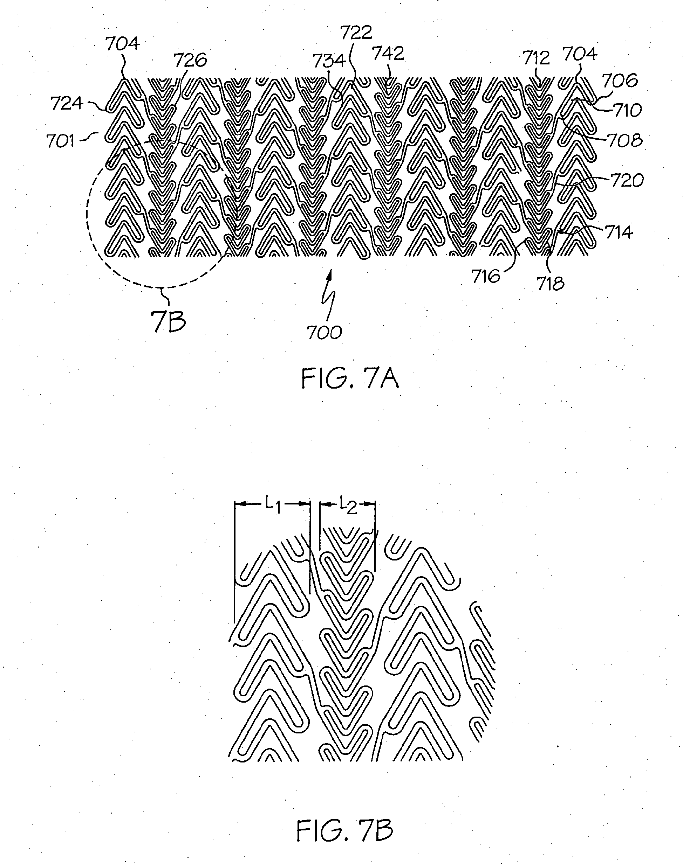

Yet another alternative embodiment of the inventive stent of the present invention is shown generally at 700 in Fig. 7a, the stent includes a plurality of circumferential bands wherein circumferential bands which are adjacent one another are connected one to the other. The circumferential bands include first circumferential bands 704 characterized by a first number of alternating first peaks 706 and first troughs 708 and second circumferential bands 712 characterized by a second number of alternating second peaks 714 and second troughs 716. The second number is different from the first number. The first peaks and troughs are oriented non-parallel to the longitudinal axis 701 of the stent 700 and the second peaks and second troughs are oriented non-parallel to the longitudinal axis 701 of the stent. Desirably, the peaks and troughs are oriented at an angle of at least 10 degrees with respect to the longitudinal axis of the stent. More desirably, the peaks and troughs are oriented at an angle of at least 15 degrees with respect to the longitudinal axis of the stent.

The first and second circumferential bands each define a pathway around the periphery of the stent and the first and second pathways are the same length.

The stent is further characterized as having bent struts exhibiting finger-like projections which are similar to those in the embodiment shown in Fig. 6a. Each of the bent struts may be characterized by a width. Optionally, the width of the bent struts of the first bands W₁ exceeds the width of the bent struts of the second bands W₂. Desirably, as shown in Fig. 7a, bent struts which are circumferentially adjacent one another are parallel to one another. Bent struts in longitudinally adjacent first and second circumferential bands may or may not be parallel to one another, however.

In the embodiment shown in Fig. 7a, first and second circumferential bands which are longitudinally adjacent one another are connected by at least one connector 720 and desirably, by a plurality of connectors. Typically, the connectors are slightly curvilinear and are non-parallel to the longitudinal axis 701 of the stent. In other embodiments of the invention, other types of connectors may be used - for example connectors with one or more curves and/or connectors of different lengths. Desirably, as shown in Fig. 7a, the connectors extend from peaks of circumferential bands to troughs of adjacent circumferential bands. Also desirably, as shown in Fig. 7a, the connectors are shorter in length than the longitudinal extent L₂ of the second circumferential bands.

Where a plurality of connectors are present between adjacent first and second circumferential bands, circumferentially adjacent connectors are joined via a first pathway along a first circumferential band and a second pathway along a second circumferential band, the first pathway desirably being of the same length as the second pathway.

In the embodiment of Fig. 7a, each first pathway traverses a total of four peaks and troughs (i.e. two peaks and two troughs) and each second pathway traverse a total of six peaks and troughs (i.e. three peaks and three troughs).

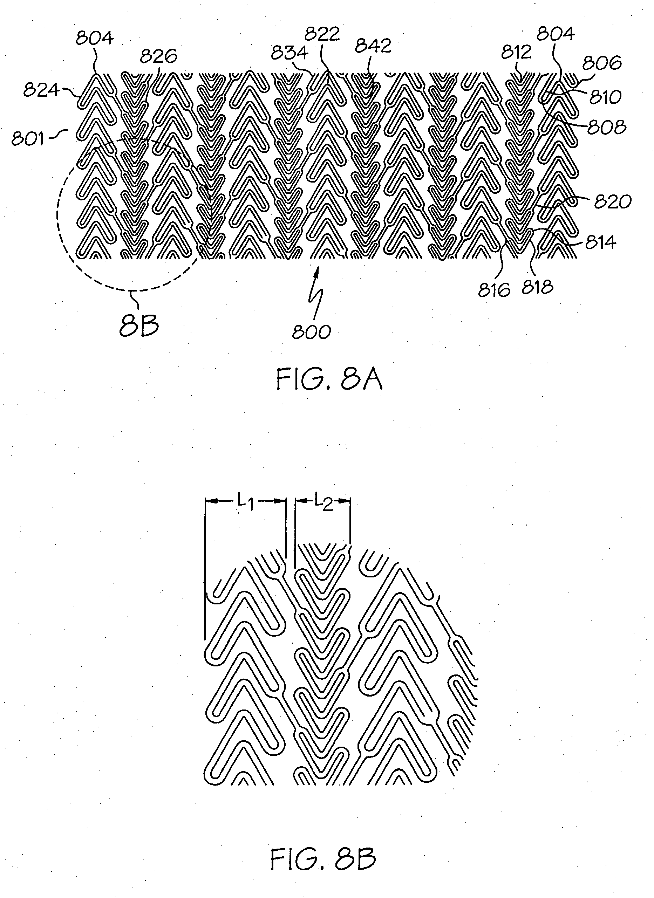

An alternative embodiment of the inventive stent of the present invention is shown in Fig. 8a. In this embodiment, the stent is substantially similar to that shown in Fig. 7a. The connectors 820 shown in the embodiment in Fig. 8a are straight while those shown in Fig. 7a are slightly curvilinear. The connectors 820 are again shorter in shorter in length than the longitudinal extent L₂ of the second circumferential bands.

Again, a plurality of connectors are shown present and between adjacent first and second circumferential bands. The circumferentially adjacent connectors are again joined via a first pathway along a first circumferential band and a second pathway along a second circumferential band, the first pathway desirably being of the same length as the second pathway. Again in the embodiment of Fig. 8a, each first pathway traverses a total of four peaks and troughs (i.e. two peaks and two troughs) and each second pathway traverse a total of six peaks and troughs (i.e. three peaks and three troughs).

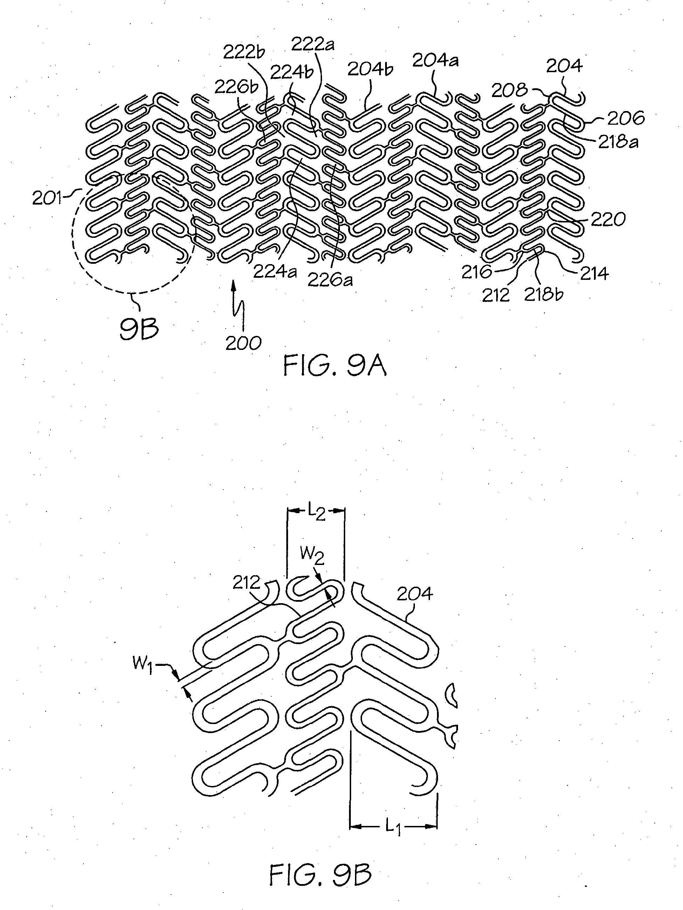

Desirably, as shown in Fig. 9b, the first and second circumferential bands 204 and 212 are each characterized by a longitudinal extent with the longitudinal extent L₁ of each the first circumferential bands exceeding the longitudinal extent L₂ of the second circumferential bands.

In yet another embodiment, as shown generally at 200 in Fig. 9a, the invention is directed to a stent including a plurality of circumferential bands where circumferential bands which are adjacent one another are connected one to the other. The circumferential bands include first circumferential bands 204 characterized by a first number of alternating first peaks 206 and first troughs 208 and second circumferential bands 212 characterized by a second number of alternating second peaks 214 and second troughs 216. The second number is different from the first number. The first peaks and troughs are oriented non-parallel to the longitudinal axis 201 of the stent and the second peaks and second troughs are oriented non-parallel to the longitudinal axis of the stent. Desirably, the peaks and troughs are oriented at an angle of at least 10 degrees with respect to the longitudinal axis of the stent. More desirably, the peaks and troughs are oriented at an angle of at least 15 degrees with respect to the longitudinal axis of the stent. Optionally, the first and second circumferential bands each define a pathway around the periphery of the stent and the first and second pathways are the same length.

Desirably, as shown in Fig. 9b, the first and second circumferential bands 204 and 212 are each characterized by a longitudinal extent with the longitudinal extent L₁ of each the first circumferential bands exceeding the longitudinal extent L₂ of the second circumferential bands.

Also desirably, first peaks and first troughs which are circumferentially adjacent one another are connected by struts 218a and second peaks and second troughs which are circumferentially adjacent one another are connected by struts 218b. Each of the struts is characterized by a width with the width W₁ of the struts of the first circumferential bands exceeding the width W₂ of the struts of the second circumferential bands.

Typically, as shown in Fig. 9a, struts which are circumferentially adjacent one another are parallel to one another.

First and second circumferential bands which are longitudinally adjacent one another may be connected by a single connector or by a plurality of connectors. The connectors may be of any shape. In one embodiment, as shown in Fig. 9a, straight connectors 220 may be used. The connectors may be oriented non-parallel to the longitudinal axis, as shown in Fig. 9b or, in another embodiment, oriented parallel to the longitudinal axis.

The connectors may extend from any region of one circumferential band to any region of an adjacent circumferential band. In the embodiment of Fig. 9a, the connectors extend from peaks of circumferential bands to troughs of adjacent circumferential bands. In the embodiment of Fig. 9a, first and second circumferential bands 204 and 212 which are longitudinally adjacent one another are connected by a plurality of connectors 220 and the connectors are shorter in length than the longitudinal extent L₂ of the second circumferential bands. Circumferentially adjacent connectors may be joined via a first pathway along a first circumferential band and a second pathway along a second circumferential band with the first pathway desirably being of the same length as the second pathway.

Desirably, as shown in Fig. 9a, the struts 218a in first circumferential bands which are longitudinally adjacent one, for example first circumferential bands 204a and 204b, are non-parallel to one another. More desirably, as shown in Fig 9a, the struts in first circumferential bands which are longitudinally adjacent one another slant in opposing directions relative to the longitudinal axis of the stent. Without being bound by theory, the alternating orientation of adjacent first circumferential bands is believed to prevent significant rotation and build-up of torque and the accompanying degradation of stent performance.

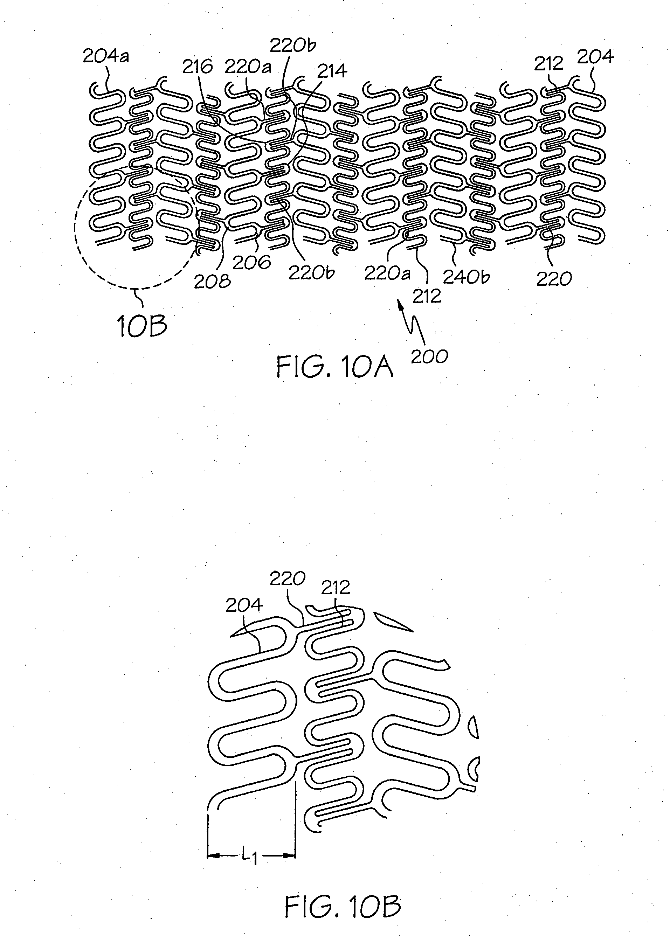

In another embodiment of the invention, the first circumferential bands are connected to the second circumferential bands via straight connectors which extend between portions of similar curvature on adjacent circumferential bands. As shown by way of example in Fig. 10a, connectors 220a extend between peaks 206 of first circumferential band 204a and peaks 214 of second circumferential band 212. Connectors 220b extend between troughs 216 of second circumferential band 212 and troughs 208 of first circumferential bands 204b.

In the embodiment of Fig. 10a, connectors 220a and 220b are shorter in length than the longitudinal extent L₁ of first circumferential bands 204 but longer than the connectors of the embodiment of Fig. 10a. In other embodiments of the invention, longer connectors may be used. Any of the other connectors disclosed herein may also be used to achieve different properties.

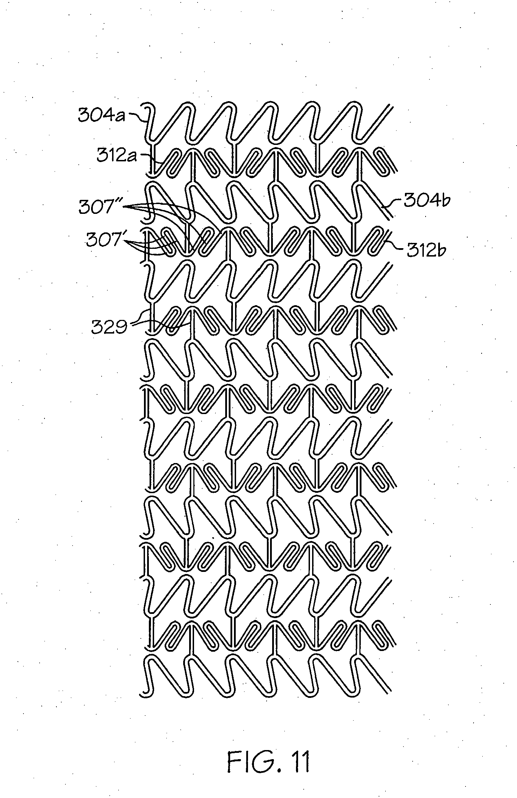

The invention is also directed to other embodiments in which the orientation of the struts in the first circumferential bands alternates between consecutive first circumferential bands relative to the longitudinal axis. In the embodiment of Fig. 11, the orientation of the struts in adjacent first circumferential bands 304a,b relative to the longitudinal axis alternates. Moreover, each second circumferential band 312a,b may comprise a plurality of consecutive struts 307' oriented in a first direction relative to the longitudinal axis of the stent and a plurality of consecutive struts 307" oriented in a second direction opposite the first direction and relative to the longitudinal axis of the stent. Adjacent circumferential bands are connected via connectors 329. Desirably, in the embodiment of Fig. 11, the connectors are oriented longitudinally. Any of the other connectors disclosed herein may be used to achieve stents with other characteristics.

First circumferential bands 304a,b may be longer or the same length about the periphery of the stent as second circumferential bands 312a,b. First circumferential bands 304a,b are desirably wider than second circumferential bands 312a,b. Also desirably, the first circumferential bands have fewer peaks and troughs than the second circumferential bands.

It is further within the scope of the invention to vary the number of connectors extending between adjacent circumferential bands in any of the embodiments disclosed herein. For example, adjacent circumferential bands in the middle of the stent may be joined by more connectors than adjacent circumferential bands at the proximal and/or distal ends of the stent so that the proximal and/or distal ends of the stent are more flexible. The middle portion of the stent may have fewer connectors than the proximal and/or distal ends of the stent to achieve greater flexibility in the middle of the stent than in the proximal and/or distal ends of the stent. The number of connectors may increase over the length of the stent to provide a stent with increasing rigidity over its length.

The invention is also directed to a stent such as that shown by way of example in Fig. 1a, comprising a sidewall with a plurality of openings 122 therein. Each opening is bounded by at least a first stent member and a second stent member. The first stent member 134 (shown shaded) is of a larger width than the second stent member 142 (shown shaded). The first stent member 134 comprises a plurality of bent first struts 110 which extend non-parallel to the longitudinal axis 101 of the stent and the second stent member 142 comprises a plurality of bent second struts 118 which extend non-parallel to the longitudinal axis of the stent. The bent first struts define finger like first projections 124 (shown shaded) which are non-parallel to the longitudinal axis of the stent and the bent second struts define finger like second projections 126 which are non-parallel to the longitudinal axis of the stent with the number of first projections exceeding the number of second projections.

Desirably, as shown in the embodiment of Fig. 1a, each opening is defined by first projections which are non-parallel to second projections.

In another embodiment, as shown by way of example in Fig. 9a, the stent comprises a plurality of openings including first openings 222a and second openings 222b. Each first opening 222a includes first projections 224a which are parallel to second projections 226a. Each second opening 222b includes first projections 224b which are non-parallel to second projections 226b.

The stent of Fig. 9a also has first openings and second openings where each first opening includes first projections which are parallel to second projections. Each second opening includes first projections which are non-parallel to second projections.

Any of the inventive stents disclosed above may be provided with a uniform diameter or may taper in portions or along the entire length of the stent. Also, the width and/or thickness of the various portions of the inventive stents may increase or decrease along a given portion of the stent. For example, the width and/or thickness of the circumferential bands and/or connectors may increase or decrease along portions of the stent or along the entire length of the stent. The amplitude and wavelength of several successive first circumferential bands may remain constant while the width and/or thickness of the successive first circumferential bands decreases. Similarly, the amplitude and wavelength of several successive second circumferential bands may remain constant while the width and/or thickness of the successive second circumferential bands decreases.

The inventive stents may also be modified so that one or both ends are more rigid or more flexible than the remainder of the stent.

The inventive stents may be manufactured using known stent manufacturing techniques. Suitable methods for manufacturing the inventive stents include laser cutting, chemical etching or stamping of a tube. The inventive stents may also be manufactured by laser cutting, chemically etching, stamping a flat sheet, rolling the sheet and, optionally, welding the sheet. Other suitable manufacturing techniques include electrode discharge machining or molding the stent with the desired design. The stent may also be manufactured by welding individual sections, for example, circumferential bands, together. Any other suitable stent manufacturing process may also be used.

Any suitable stent material may be used in the manufacture of the inventive stents. Examples of such materials include polymeric materials, metals, ceramics and composites. Suitable polymeric materials include thermotropic liquid crystal polymers (LCP's). Where the stent is made of metal, the metal may be stainless steel, cobalt chrome alloys such as elgiloy, tantalum or other plastically deformable metals. Other suitable metals include shape-memory metals such as nickel-titanium alloys generically known as "nitinol", platinum/tungsten alloys and titanium alloys.

The invention also contemplates the use of more than one material in the inventive stents. For example, the first undulating bands and the second undulating bands may be made of different materials. Optionally, the connectors may be made of a different material than the first and/or second undulating bands.

The inventive stents may be provided in mechanically expandable form, in self-expanding form or as a hybrid of the two. Mechanically expandable stents, in accordance with the invention, may be expanded using any suitable mechanical device including a balloon.

The inventive stents may include suitable radiopaque coatings. For example, the stents may be coated with gold or other noble metals or sputtered with tantalum or other metals. The stents may also be made directly from a radiopaque material to obviate the need for a radiopaque coating or may be made of a material having a radiopaque inner core. Other radiopaque metals which may be used include platinum, platinum-tungsten, palladium, platinum-iridium, rhodium, tantalum, or alloys or composites of these metals.

The inventive stents may also be provided with various bio-compatible coatings to enhance various properties of the stent. For example, the inventive stents may be provided with lubricious coatings. The inventive stents may also be provided with drug-containing coatings which release drugs over time. The increased surface area of a stent having bent struts provides for increased drug coatability. The bent struts also provide for point contact with a crimper versus strut/strut contact. Less contact with the crimper results in less disruption of the drug coating.

The inventive stents may also be provided with a sugar or more generally a carbohydrate and/or a gelatin to maintain the stent on a balloon during delivery of the stent to a desired bodily location. Other suitable compounds for treating the stent include biodegradable polymers and polymers which are dissolvable in bodily fluids. Portions of the interior and/or exterior of the stent may be coated or impregnated with the compound. Mechanical retention devices may also be used to maintain the stent on the balloon during delivery. To that end, the use of other coatings on the inventive stents is also within the scope of the invention.

The coating may comprise one or more non-genetic therapeutic agents, genetic materials and cells and combinations thereof as well as other polymeric coatings.

Non-genetic therapeutic agents include anti-thrombogenic agents such as heparin, heparin derivatives, urokinase, and PPack (dextrophenylalanine proline arginine chloromethylketone); anti-proliferative agents such as enoxaprin, angiopeptin, or monoclonal antibodies capable of blocking smooth muscle cell proliferation, hirudin, and acetylsalicylic acid; anti-inflammatory agents such as dexamethasone, prednisolone, corticosterone, budesonide, estrogen, sulfasalazine, and mesalamine; antineoplastic/antiproliferative/anti-miotic agents such as paclitaxel, 5-fluorouracil, cisplatin, vinblastine, vincristine, epothilones, endostatin, angiostatin and thymidine kinase inhibitors; anesthetic agents such as lidocaine, bupivacaine, and ropivacaine; anticoagulants such as D-Phe-Pro-Arg chloromethyl keton, an RGD peptide-containing compound, heparin, antithrombin compounds, platelet receptor antagonists, anti-thrombin anticodies, anti-platelet receptor antibodies, aspirin, prostaglandin inhibitors, platelet inhibitors and tick antiplatelet peptides; vascular cell growth promotors such as growth factor inhibitors, growth factor receptor antagonists, transcriptional activators, and translational promotors; vascular cell growth inhibitors such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin; cholesterol-lowering agents; vasodilating agents; and agents which interfere with endogenous vascoactive mechanisms.

Genetic materials include anti-sense DNA and RNA, DNA coding for, anti-sense RNA, tRNA or rRNA to replace defective or deficient endogenous molecules, angiogenic factors including growth factors such as acidic and basic fibroblast growth factors, vascular endothelial growth factor, epidermal growth factor, transforming growth factor α and β, platelet-derived endothelial growth factor, platelet-derived growth factor, tumor necrosis factor α, hepatocyte growth factor and insulin like growth factor, cell cycle inhibitors including CD inhibitors, thymidine kinase ("TK") and other agents useful for interfering with cell proliferation the family of bone morphogenic proteins ("BMP's"),BMP-2, BMP-3, BMP-4, BMP-5, BMP-6 (Vgr-1), BMP-7 (OP-1), BMP-8, BMP-9, BMP-10, BMP-11, BMP-12, BMP-13, BMP-14, BMP-15, and BMP-16. Desirable BMP's are any of BMP-2, BMP-3, BMP-4, BMP-5, BMP-6 and BMP-7. These dimeric proteins can be provided as homodimers, heterodimers, or combinations thereof, alone or together with other molecules. Alternatively or, in addition, molecules capable of inducing an upstream or downstream effect of a BMP can be provided. Such molecules include any of the "hedgehog" proteins, or the DNA's encoding them.

Cells can be of human origin (autologous or allogeneic) or from an animal source (xenogeneic), genetically engineered if desired to deliver proteins of interest at the transplant site. The cells may be provided in a delivery media. The delivery media may be formulated as needed to maintain cell function and viability.

Suitable polymer coating materials include polycarboxylic acids, cellulosic polymers, including cellulose acetate and cellulose nitrate, gelatin, polyvinylpyrrolidone, cross-linked polyvinylpyrrolidone, polyanhydrides including maleic anhydride polymers, polyamides, polyvinyl alcohols, copolymers of vinyl monomers such as EVA, polyvinyl ethers, polyvinyl aromatics, polyethylene oxides, glycosaminoglycans, polysaccharides, polyesters including polyethylene terephthalate, polyacrylamides, polyethers, polyether sulfone, polycarbonate, polyalkylenes including polypropylene, polyethylene and high molecular weight polyethylene, halogenated polyalkylenes including polytetrafluoroethylene, polyurethanes, polyorthoesters, proteins, polypeptides, silicones, siloxane polymers, polylactic acid, polyglycolic acid, polycaprolactonc, polyhydroxybutyrate valerate and blends and copolymers thereof, coatings from polymer dispersions such as polyurethane dispersions (for example, BAYHDROL®), fibrin, collagen and derivatives thereof, polysaccharides such as celluloses, starches, dextrans, alginates and derivatives, hyaluronic acid, squalene emulsions. Polyacrylic acid, available as HYDROPLUS® (Boston Scientific Corporation, Natick, Mass.), and described in U.S. Pat. No. 5,091,205, the disclosure of which is particularly desirable. Even more desirable is a copolymer of polylactic acid and polycaprolactone.

The inventive stents may also be used as the framework for a graft. Suitable coverings include nylon, collagen, PTFE and expanded PTFE, polyethylene terephthalate and KEVLAR, or any of the materials disclosed in US 5,824,046 and US 5,755,770. More generally, any known graft material may be used including synthetic polymers such as polyethylene, polypropylene, polyurethane, polyglycolic acid, polyesters, polyamides, their mixtures, blends and copolymers.

The inventive stents may find use in coronary arteries, renal arteries, peripheral arteries including iliac arteries, arteries of the neck and cerebral arteries. The stents of the present invention, however, arc not limited to use in the vascular system and may also be advantageously employed in other body structures, including but not limited to arteries, veins, biliary ducts, urethras, fallopian tubes, bronchial tubes, the trachea, the esophagus and the prostate.

Suitable stent delivery devices such as those disclosed in US 6,123,712, US 6,120,522 and US 5,957,930 may be used to deliver the inventive stents to the desired bodily location. The choice of delivery device will depend on whether a self-expanding or balloon expandable stent is used. The inventive stents may be delivered in conjunction with one or more stent retaining sleeves. An example of stent retaining sleeves is disclosed in US provisional application 60/238178.

The above disclosure is intended to be illustrative and not exhaustive. This description will suggest many variations and alternatives to one of ordinary skill in this art. All these alternatives and variations are intended to be included within the scope of the claims where the term "comprising" means "including, but not limited to". Those familiar with the art may recognize other equivalents to the specific embodiments described herein which equivalents are also intended to be encompassed by the claims.

Further, the particular features presented in the dependent claims can be combined with each other in other manners within the scope of the invention such that the invention should be recognized as also specifically directed to other embodiments having any other possible combination of the features of the dependent claims. For instance, for purposes of claim publication, any dependent claim which follows should be taken as alternatively written in a multiple dependent form from all prior claims which possess all antecedents referenced in such dependent claim if such multiple dependent format is an accepted format within the jurisdiction (e.g. each claim depending directly from claim 1 should be alternatively taken as depending from all previous claims). In jurisdictions where multiple dependent claim formats are restricted, the following dependent claims should each be also taken as alternatively written in each singly dependent claim format which creates a dependency from a prior antecedent-possessing claim other than the specific claim listed in such dependent claim below (e.g. claim 3 may be taken as alternatively dependent from claim 2; claim 4 may be taken as alternatively dependent on claim 2, or on claim 3, claim 5 may be taken as alternatively dependent on claim 1, claim 2, or on claim 3; etc.).

## Claims

1. A stent comprising a plurality of circumferential bands (104, 112), circumferential bands (104, 112) which are adjacent one another connected one to the other, the circumferential bands (104, 112) including first circumferential bands (104) comprising a first number of alternating first peaks (106) and first troughs (108) joined by struts (110) and second circumferential bands (112) comprising a second number of alternating second peaks (114) and second troughs (116) joined by struts (118), wherein the second number is different from the first number, **characterized in that**
the struts (110) of the first circumferential bands (104) and the struts (118) of the second circumferential bands (112) are bent and
a width (W₁) of the bent struts (110) of the first bands (104) exceeds a width (W₂) of the bent struts (118) of the second bands (112).

2. The stent of claim 1 wherein the first and second circumferential bands (104, 112) are each **characterized by** a longitudinal extent (L₁, L₂), the longitudinal extent (L₁) of each first circumferential band (104) exceeding the longitudinal extent (L₂) of each second circumferential band (112).

3. The stent of claim 1 wherein bent struts (110, 118) which are circumferentially adjacent one another are parallel to one another.

4. The stent of claim 3 wherein the bent struts (110, 118) in longitudinally adjacent first and second circumferential bands (104, 112) are non-parallel to one another.

5. The stent of claim 3 wherein the first and second circumferential bands (104, 112) are each **characterized by** a longitudinal extent, the longitudinal extent of each first circumferential band (104) exceeding the longitudinal extent of the second circumferential bands (112).

6. The stent of claim 5 wherein first and second circumferential bands (104, 112) which are longitudinally adjacent one another are connected by a plurality of connectors (120).

7. The stent of claim 6 wherein the connectors (120) are straight.

8. The stent of claim 6 wherein the connectors (120) are non-parallel to the longitudinal axis of the stent.

9. The stent of claim 7 wherein the connectors (120) extend from peaks (114) of circumferential bands to troughs (116) of adjacent circumferential bands.

10. The stent of claim 7 wherein first and second circumferential bands (104, 112) which are longitudinally adjacent one another are connected by a plurality of connectors (120), the connectors (120) being shorter in length than the longitudinal extent of the second circumferential bands (112).

11. The stent of claim 6 wherein circumferentially adjacent connectors (120) are joined via a first pathway along a first circumferential band (104) and a second pathway along a second circumferential band (104), the first pathway being of the same length as the second pathway.

12. The stent of claim 11 wherein each first pathway traverses a total of three peaks (114) and troughs (116) and each second pathway traverse a total of five peaks (114) and troughs (116).

13. The stent of claim 1 wherein the first and second circumferential bands (104, 112) each define a pathway around the periphery of the stent, the first and second pathways being of the same length.

14. The stent of claim 1 wherein the first and second peaks (106) and first and second troughs (108) are oriented at an angle between 0° and 70° with respect to the longitudinal axis of the stent.

15. The stent of claim 1 wherein the first and second peaks (106) and first and second troughs (108) are oriented at an angle of at least 15 degrees with respect to the longitudinal axis of the stent.

## Patentansprüche

1. Stent, umfassend eine Vielzahl von umlaufenden Bändern (104, 112), wobei umlaufende Bänder (104, 112), die aneinander grenzen miteinander verbunden sind, die umlaufenden Bänder (104, 112) erste umlaufende Bänder (104) umfassend eine erste Anzahl von alternierenden ersten Gipfeln (106) und ersten Wannen (108), die durch Streben (110) verbunden sind beinhalten, sowie zweite umlaufende Bänder (112), umfassend eine zweite Anzahl von alternierenden ersten Gipfeln (114) und zweiten Wannen (116), die durch Streben (118) verbunden sind, wobei die zweite Anzahl von der ersten Anzahl verschieden ist, **dadurch gekennzeichnet dass** die Streben (110) der ersten umlaufenden Bänder (104) und die Streben (118) der zweiten umlaufenden Bänder (112) gebogen sind und
eine Breite (W₁) der gebogenen Streben (110) der ersten Bänder (104) eine Breite (W₂) der gebogenen Streben (118) der zweiten Bänder (112) übertrifft.

2. Stent gemäß Anspruch 1, wobei die ersten und zweiten umlaufenden Bänder (104, 112) jeweils durch eine Längserstreckung (L₁, L₂) gekennzeichnet sind, wobei die Längserstreckung (L₁) von jedem ersten umlaufenden Band (104) die Längserstreckung (L₂) von jedem zweiten umlaufenden Band (112) übertrifft.

3. Stent gemäß Anspruch 1, wobei gebogene Streben (110, 118), die in Umfangsrichtung aneinander grenzen, parallel zueinander sind.

4. Stent gemäß Anspruch 3, wobei die gebogenen Streben (110, 118) in ersten und zweiten umlaufenden Bändern (104, 112), die in Längsrichtung aneinander grenzen, nicht parallel zueinander sind.

5. Stent gemäß Anspruch 3, wobei die ersten und zweiten umlaufenden Bänder (104, 112) jeweils durch eine Längserstreckung gekennzeichnet sind, wobei die Längserstreckung von jedem ersten umlaufenden Band (104) die Längserstreckung der zweiten umlaufenden Bänder (112) übertrifft.

6. Stent gemäß Anspruch 5, wobei erste und zweite umlaufende Bänder (104, 112), die in Längsrichtung aneinander grenzen, durch eine Vielzahl von Verbindern (120) verbunden sind.

7. Stent gemäß Anspruch 6, wobei die Verbinder (120) gerade sind.

8. Stent gemäß Anspruch 6, wobei die Verbinder (120) zu der Längsachse des Stents nicht parallel sind.

9. Stent gemäß Anspruch 7, wobei die Verbinder (120) sich von Gipfeln (114) umlaufender Bänder zu Wannen (116) von aneinander grenzenden umlaufender Bändern erstrecken.

10. Stent gemäß Anspruch 7, wobei erste und zweite umlaufende Bänder (104, 112), die in Längsrichtung aneinander grenzen, durch eine Vielzahl von Verbindern (120) verbunden sind und die Verbinder (120) in der Länge kürzer sind als die Längserstreckung der zweiten umlaufenden Bänder (112).

11. Stent gemäß Anspruch 6, wobei in Umlaufsrichtung aneinander grenzende Verbinder (120) über einen ersten Weg entlang eines ersten umlaufenden Bandes (104) und einen zweiten Weg entlang eines zweiten umlaufenden Bandes (104) verbunden sind und der erste Weg von gleicher Länge ist wie der zweite Weg.

12. Stent gemäß Anspruch 11, wobei jeder erste Weg eine Gesamtmenge von drei Gipfeln (114) und Wannen (116) durchquert, und jeder zweite Weg eine Gesamtmenge von fünf Gipfeln (114) und Wannen (116) durchquert.

13. Stent gemäß Anspruch 1, wobei die ersten und zweiten umlaufenden Bänder (104, 112) jeweils einen Weg um die Peripherie des Stents herum definieren und die ersten und zweiten Wege von gleicher Länge sind.

14. Stent gemäß Anspruch 1, wobei die ersten und zweiten Gipfel (106) und die ersten und zweiten Wannen (108) in Bezug auf die Längsachse des Stents in einem Winkel zwischen 0° und 70° ausgerichtet sind.

15. Stent gemäß Anspruch 1, wobei die ersten und zweiten Gipfel (106) und die ersten und zweiten Wannen (108) in Bezug auf die Längsachse des Stents in einem Winkel von mindestens 15 Grad ausgerichtet sind.

## Revendications

1. Stent, comportant une pluralité de bandes circonférentielles (104, 112), dans lequel des bandes circonférentielles (104, 112) adjacentes l'une à l'autre sont reliées l'une à l'autre, les bandes circonférentielles (104, 112) incluent premières bandes circonférentielles (104) comportant un premier nombre de premiers sommets (106) et de premières vallées (108) alternantes, qui sont reliés par des contrefiches (110), et deuxièmes bandes circonférentielles (112) comportant un deuxième nombre de deuxièmes sommets (114) et deuxièmes vallées (116) alternantes qui sont reliés par des contrefiches (118), le deuxième nombre étant différent du premier nombre, **caractérisé en ce que**
les contrefiches (110) des premières bandes circonférentielles (104) et les contrefiches (118) des deuxièmes bandes circonférentielles (112) sont courbées et
une largeur (W₁) des contrefiches courbées (110) des premières bandes (104) surmonte une largeur (W₂) des contrefiches courbées (118) des deuxièmes bandes (112).

2. Stent selon la revendication 1, dans lequel les premiers et deuxièmes bandes circonférentielles (104, 112) sont chacune **caractérisée par** une extension longitudinale (L₁, L₂) l'extension longitudinale (L₁) de chaque première bande circonférentielle (104) surmontant 1' extension longitudinale (L₂) de chaque deuxième bande circonférentielle (112).

3. Stent selon la revendication 1, dans lequel des contrefiches courbées (110, 118), qui sont adjacentes l'une à l'autre dans la direction circonférentielle, sont parallèles l'une à l'autre.

4. Stent selon la revendication 3, dans lequel les contrefiches courbées (110, 118) dans premières et deuxièmes bandes circonférentielles (104, 112) adjacentes dans la direction longitudinale, ne sont pas parallèles l'une à l'autre.

5. Stent selon la revendication 3, dans lequel les premières et deuxièmes bandes circonférentielles (104, 112) sont chacune **caractérisée par** une extension longitudinale, l'extension longitudinale de chaque première bande circonférentielle (104) surmontant l'extension longitudinale des deuxièmes bandes circonférentielles (112).

6. Stent selon la revendication 5, dans lequel premières et deuxième bandes circonférentielles (104, 112), qui sont adjacentes l'une à l'autre dans la direction longitudinale, sont reliées par une pluralité de connecteurs (120).

7. Stent selon la revendication 6, dans lequel les connecteurs (120) sont droites.

8. Stent selon la revendication 6, dans lequel les connecteurs (120) ne sont pas parallèles à l'axe longitudinal du Stent.

9. Stent selon la revendication 7, dans lequel les connecteurs (120) s'étendent de sommets (114) de bandes circonférentielles à des vallées (116) de bandes circonférentielles adjacentes .

10. Stent selon la revendication 7, dans lequel premières et deuxièmes bandes circonférentielles (104, 112), adjacentes l'une à l'autre dans la direction longitudinale, sont reliées par une pluralité de connecteurs (120), et les connecteurs (120) sont plus courts en longueur que l'extension longitudinale des deuxièmes bandes circonférentielles (112).

11. Stent selon la revendication 6, dans lequel connecteurs (120) qui sont adjacents dans le sens circonférentiel sont reliés à travers un premier chemin le long d'une première bande circonférentielle (104), et à travers un deuxième chemin le long d'une deuxième bande circonférentielle (104), le première chemin ayant la même longueur comme le deuxième chemin.

12. Stent selon la revendication 11, dans lequel chaque premier chemin traverse un total de trois sommets (114) et vallées (116), et chaque deuxième chemin traverse un total de cinq sommets (114) et vallées (116).

13. Stent selon la revendication 1, dans lequel les premières et deuxièmes bandes circonférentielles (104, 112) chacune définissent un chemin autour de la périphérie du Stent, et les premiers et deuxièmes chemins ont la même longueur.

14. Stent selon la revendication 1, dans lequel les premiers et deuxièmes sommets (106) et les premières et deuxièmes vallées (108) sont orientés sous un angle compris entre 0° et 70° par rapport à l'axe longitudinal du Stent.

15. Stent selon la revendication 1, dans lequel les premiers et deuxièmes sommets (106) et les premières et deuxièmes vallées (108) sont orientés sous un angle d'au moins 15 degrés par rapport à l'axe longitudinal du Stent.
